# FASCICULE DE BREVET EUROPEEN

(11) **EP 3 716 898 B1**
(45) Date de publication et mention de la délivrance du brevet: **08.04.2026**
(21) Numéro de dépôt: 18819544.0
(22) Date de dépôt: 27.11.2018
(51) Int. Cl.: A61F 2/00, A61F 5/00

(54) **MANCHETTE OCCLUSIVE ET SYSTEME OCCLUSIF IMPLANTABLE COMPRENANT UNE TELLE MANCHETTE**
OKKLUSIONSMANSCHETTE UND IMPLANTIERBARES OKKLUSIONSSYSTEM MIT SOLCH EINER MANSCHETTE
OCCLUSION COLLAR AND IMPLANTABLE OCCLUSION SYSTEM COMPRISING SUCH A COLLAR

(30) Priorité: 28.11.2017 FR 1761287
(43) Date de publication de la demande: 07.10.2020
(73) Titulaire: Uromems, 38320 Eybens (FR)
(72) Inventeur: LAMRAOUI, Hamid, 38000 Grenoble (FR); MASRI, Christopher, 38000 Grenoble (FR)
(74) Mandataire: Regimbeau
(86) Numéro de dépôt international: PCT/FR2018/052985
(87) Numéro de publication internationale: WO 2019/106274

(56) Documents cités:
- EP-A1- 2 815 720
- WO-A1-2005/009293
- WO-A1-2013/053929
- WO-A1-2013/101764
- WO-A1-2013/165563
- US-A1- 2015 230 904

## Description

### DOMAINE DE L'INVENTION

La présente invention concerne une manchette occlusive destinée à obturer sélectivement un conduit anatomique, tel qu'un urètre ou un col vésical, ainsi qu'un système occlusif implantable comprenant une telle manchette.

### ETAT DE LA TECHNIQUE

Il existe des systèmes occlusifs implantables destinés à obturer sélectivement un conduit anatomique, par exemple pour remédier à une incontinence (cas des sphincters artificiels urinaires et anaux) ou pour limiter l'entrée d'aliments dans l'estomac (cas des bandes gastriques). L'obturation du conduit anatomique est engendrée par la compression exercée par une manchette enroulée autour dudit conduit.

Parmi les manchettes, on s'intéresse dans le présent texte à celles basées sur une technologie fluidique (la manchette comprenant un réservoir gonflable couplé à un circuit fluidique permettant de remplir ou vider sélectivement ledit réservoir, selon la compression à exercer sur le conduit anatomique).

Parmi les systèmes occlusifs, certains sont manuels, c'est-à-dire que c'est un utilisateur (par exemple le patient lui-même) qui commande la compression appliquée par la manchette. D'autres systèmes sont automatiques, c'est-à-dire qu'ils comprennent un ou plusieurs capteurs, un actionneur et une unité de contrôle qui commande l'actionneur pour imposer une compression déterminée du conduit anatomique par la manchette, sans qu'une intervention d'un utilisateur ne soit nécessaire.

Le document WO 2016/083428 décrit un système occlusif implantable automatique. Ce système comprend un circuit fluidique comprenant :
- une manchette occlusive gonflable contenant un volume variable d'un fluide, destinée à entourer au moins une partie d'un conduit naturel à occlure,
- un récipient à volume variable rempli d'un fluide, ledit récipient comprenant une partie fixe et une partie mobile,
- une liaison fluidique entre le récipient et la manchette occlusive,

Par ailleurs, ce système comprend un actionneur couplé mécaniquement à la partie mobile du récipient de sorte à déplacer linéairement ladite partie mobile par rapport à la partie fixe pour ajuster le volume du récipient.

Aussi, le document WO 2013 101 764 décrit: une manchette occlusive comprenant : une bande adaptée pour entourer un conduit anatomique, pourvue d'un dispositif de fermeture adapté pour maintenir la bande enroulée sur elle-même sur une longueur déterminée, un réservoir gonflable agencé sur une face intérieure de la bande, et un connecteur agencé à une extrémité de la bande pour assurer une liaison fluidique entre le réservoir et une tubulure.

L'actionneur et le récipient à volume variable sont agencés dans un boîtier étanche contenant un gaz.

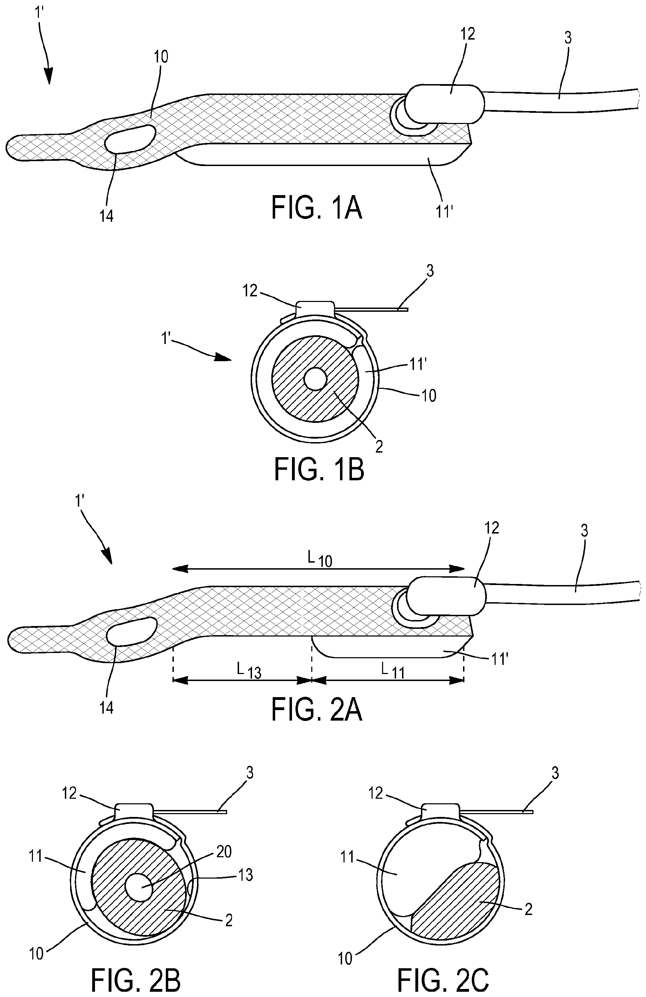

Les figures 1A et 1B sont des vues d'une manchette occlusive 1' utilisable dans ce système, respectivement à l'état libre (avant l'implantation), et une fois implantée autour du conduit anatomique 2 à obturer.

Ladite manchette comprend une bande 10 adaptée pour entourer ledit conduit anatomique, et un réservoir gonflable 11' agencé sur une face de la bande 10, sensiblement sur toute la longueur de celle-ci. Le réservoir gonflable 11 comprend à l'une de ses extrémités un connecteur 12 permettant d'assurer une liaison fluidique entre l'intérieur du réservoir 11 et une tubulure 3 reliée au récipient.

A son extrémité opposée audit connecteur 12, la bande présente une ouverture oblongue 14, qui est destinée à être engagée sur le connecteur 12 afin de maintenir la manchette en position enroulée autour du conduit anatomique 2.

Dans le présent texte, on entend par « longueur » de la manchette la circonférence intérieure de la manchette lorsque celle-ci est dans sa position fermée. Cette longueur est notée L₁₀ sur la figure 2A. Selon le dispositif de fermeture de la manchette, cette longueur est donc généralement inférieure à la longueur totale de la bande.

Il existe des manchettes de différentes longueurs, selon les applications et patients visés.

La longueur de la manchette occlusive est choisie par le chirurgien, en fonction de la circonférence du conduit anatomique à l'emplacement prévu pour la manchette.

Dans le cas des sphincters urinaires artificiels, la manchette est généralement implantée chez la femme autour du col vésical, qui présente une circonférence importante. Typiquement, la longueur de la manchette pour une telle application est de l'ordre d'une dizaine de centimètres.

Cependant, l'emploi d'une manchette de cette dimension pose un certain nombre de problèmes pour l'implantation du système et son fonctionnement.

Un premier inconvénient d'une telle manchette est son encombrement. Or, l'implantation d'une telle manchette autour du col vésical de la femme est délicate car il y a peu d'espace autour du col vésical pour insérer la manchette. En outre, l'emplacement de la manchette est très proche de la paroi vaginale, que le chirurgien doit veiller à ne pas perforer pendant l'implantation.

D'une part, le volume de la manchette étant important, le récipient de fluide doit être dimensionné en conséquence pour procurer une compression suffisante du conduit anatomique par la manchette. Ceci implique une augmentation de l'encombrement du boîtier contenant ledit récipient.

Par ailleurs, la variation de volume du récipient, qui est mise en oeuvre avec une amplitude importante, entraîne également une réduction de l'autonomie de la source d'énergie qui alimente l'actionneur, lorsque celle-ci est également agencée dans le boîtier.

Par conséquent, l'emploi d'une manchette de grande dimension implique à la fois une plus grande complexité de l'opération d'implantation, une augmentation du volume du boîtier implantable et une diminution de l'autonomie du système.

### EXPOSE DE L'INVENTION

Un but de l'invention est donc de concevoir une manchette occlusive qui puisse être facilement implantée autour d'un conduit anatomique présentant une circonférence importante, sans engendrer les problèmes susmentionnés.

A cet effet, l'invention propose une manchette occlusive pour obturer sélectivement un conduit anatomique, comprenant :
- une bande adaptée pour entourer ledit conduit anatomique, pourvue d'un dispositif de fermeture adapté pour maintenir la bande enroulée sur elle-même sur une longueur déterminée, et
- un réservoir gonflable agencé sur une face intérieure de la bande.

Ladite manchette est caractérisée en ce que le réservoir gonflable s'étend sur seulement une partie de la longueur de la bande, une autre partie de ladite longueur définissant une surface intérieure libre de la bande configurée pour former une zone d'appui pour le conduit anatomique, de sorte que lorsque la bande est maintenue enroulée sur elle-même par le dispositif de fermeture, le réservoir gonflable est en vis-à-vis de ladite surface intérieure libre de la bande.

Conformément à son acception courante, le terme « libre » signifie dans le présent texte que la surface intérieure de la bande ne supporte aucun élément additionnel, lequel serait susceptible de s'interposer entre la bande et le conduit anatomique.

Dans le présent texte, le terme « intérieur » désigne la face de la manchette qui, lorsque la manchette est en position fermée, est destinée à venir en contact direct avec le conduit anatomique. Le terme « extérieur » désigne la face opposée.

De manière particulièrement avantageuse, la longueur du réservoir est choisie de telle sorte que lorsque la bande est enroulée sur elle-même autour du conduit anatomique et que le réservoir est gonflé, le conduit anatomique est comprimé entre la paroi du réservoir et la surface libre de la bande, le réservoir exerçant un effort de poussée du conduit anatomique contre ladite surface libre de la bande.

Le réservoir gonflable s'étend de préférence sur une longueur inférieure ou égale à 75% de la longueur de la bande.

La longueur de la bande peut être comprise entre 30 et 110 mm, de préférence entre 70 et 110 mm.

Selon un mode de réalisation, la bande est formée d'un textile enrobé d'un élastomère biocompatible. Le réservoir gonflable peut être réalisé en un élastomère biocompatible. Selon un mode de réalisation, ledit élastomère biocompatible est du silicone.

De manière avantageuse, la manchette comprend en outre un connecteur agencé à une extrémité de la bande pour assurer une liaison fluidique entre le réservoir et une tubulure.

Selon un mode de réalisation, le dispositif de fermeture comprend une ouverture ménagée à une extrémité de la bande et apte à s'engager autour du connecteur pour maintenir la bande enroulée sur elle-même.

De manière alternative, le dispositif de fermeture comprend une surface pourvue de crans à une extrémité de la bande et un dispositif d'attache comprenant une fente pour l'insertion de ladite extrémité, configuré pour bloquer les crans à l'encontre d'un effort d'extraction de ladite extrémité. De préférence, ces crans sont agencés sur la face extérieure de la bande.

Un autre objet de l'invention concerne un système occlusif implantable comprenant une manchette telle que décrite plus haut et un boîtier implantable comprenant une pompe en liaison fluidique avec la manchette pour faire varier le volume de fluide dans le réservoir gonflable.

Selon une application particulière de l'invention, le système occlusif est un sphincter urinaire artificiel.

### DESCRIPTION DES FIGURES

D'autres caractéristiques et avantages de l'invention ressortiront de la description détaillée qui va suivre, en référence aux dessins annexés sur lesquels :
- la figure 1A est une vue schématique d'une manchette occlusive de type connu, telle que fournie au chirurgien en vue de l'implantation ;
- la figure 1B est une vue schématique de la manchette de la figure 1A, après implantation autour du conduit anatomique (représentée comme n'exerçant pas de compression sur ledit conduit) ;
- la figure 2A est une vue schématique d'une manchette occlusive selon un mode de réalisation de l'invention, telle que fournie au chirurgien en vue de l'implantation ;
- la figure 2B est une vue schématique de la manchette de la figure 2A, après implantation autour du conduit anatomique (représentée comme n'exerçant pas de compression sur ledit conduit) ;
- la figure 2C est une vue schématique de la manchette de la figure 2A, après implantation autour du conduit anatomique, en configuration d'obturation dudit conduit ;
- la figure 3 illustre une variante du système de fermeture de la manchette occlusive ;
- la figure 4A illustre une simulation par éléments finis de la contrainte compressive générée dans un col vésical par la compression exercée par une manchette telle qu'illustrée sur la figure 1A ;
- la figure 4B illustre une simulation par éléments finis de la contrainte compressive générée dans le col vésical par la compression exercée par une manchette selon un mode de réalisation de l'invention ;
- la figure 5 illustre un système occlusif implantable comprenant une manchette selon un mode de réalisation de l'invention.

Les signes de référence identiques d'une figure à l'autre désignent des éléments identiques ou tout au moins remplissant la même fonction.

### DESCRIPTION DETAILLEE DE MODES DE REALISATION DE L'INVENTION

Par rapport aux manchettes occlusives connues, l'invention propose de réduire la longueur du réservoir gonflable.

Les figures 2A-2C illustrent de manière schématique une manchette 1 selon un mode de réalisation de l'invention, respectivement à l'état libre, à l'état fermé sans exercer de compression sur le conduit et dans une configuration d'obturation dudit conduit.

Le réservoir gonflable 11 s'étend sur moins de 75% de la longueur L₁₀ de la bande, de préférence moins de 60% de la longueur de la bande, et de manière encore préférée moins de 50% de la longueur de la bande. La longueur du réservoir gonflable est notée L₁₁.

Ainsi, par exemple, pour une manchette de 10 cm de long, la longueur du réservoir gonflable est de 4,5 cm.

En d'autres termes, la face intérieure de la bande 10 se décompose en une région de longueur L₁₁ sur laquelle est agencé le réservoir 11, et une région de longueur L₁₃ qui est forme une surface libre, c'est-à-dire dépourvue de réservoir, et qui constitue une zone d'appui pour le conduit anatomique.

Lorsque la manchette est en position fermée, la paroi du réservoir 11 se trouve donc en regard de la surface libre 13 de la bande (le conduit anatomique 2 étant interposé entre ces deux surfaces, comme le montre la figure 2B).

Au contraire, dans une manchette de type connu telle que représentée sur les figures 1A et 1B, les deux extrémités du réservoir sont jointives lorsque la manchette est fermée. Même s'il peut subsister un léger jeu entre ces deux extrémités, notamment pour tenir compte de la variation de volume du réservoir lors de son gonflement, la zone correspondante de la bande ne constitue pas une zone d'appui pour le conduit anatomique.

Revenant à l'invention, lorsque l'on gonfle le réservoir 11, celui-ci pousse le conduit anatomique contre la surface libre 13 de la bande, obturant ainsi progressivement le conduit (cf. figure 2C). Ainsi, le conduit anatomique est comprimé entre, d'une part, le réservoir 11 et, d'autre part, la surface libre 13.

De manière avantageuse, on met à disposition du chirurgien une panoplie de manchettes de longueurs différentes, parmi lesquelles le chirurgien choisira la plus adaptée au patient et à l'emplacement prévu pour l'implantation. Dans cette panoplie, la longueur de la bande est généralement comprise entre 3 et 11 cm.

Pour les manchettes de petite longueur (c'est-à-dire typiquement inférieure à 7 cm), le réservoir gonflable peut être agencé sur toute la longueur de la manchette.

A partir d'une certaine longueur de manchette (par exemple 7 cm), la longueur du réservoir gonflable peut être fixe (par exemple de l'ordre de 4,5 cm). Ainsi, on peut utiliser la même pompe pour toutes les dimensions de manchette, ce qui évite de multiplier les références de boîtiers.

Une telle manchette peut être fabriquée de la manière suivante.

D'une part, la bande est formée d'un textile enrobé d'un élastomère biocompatible. De manière avantageuse, le textile est un tissu de polyester biocompatible, connu notamment sous le nom de DACRON^{™} et l'élastomère est du silicone. La bande peut être découpée, par exemple par jet d'eau, à partir d'une feuille de grandes dimensions. La bande ainsi formée présente l'avantage d'être lisse, de ne pas s'enchâsser dans les tissus anatomiques (autorisant ainsi une éventuelle explantation de la manchette) et de ne pas être étirable.

D'autre part, le réservoir est réalisé en un élastomère biocompatible, par exemple du silicone, de préférence par trempage de manière à assurer un état de surface parfaitement lisse afin d'assurer un contact souple avec le conduit anatomique.

Le réservoir est collé sur la bande.

Le connecteur est quant à lui moulé et collé sur la face de la bande opposée au réservoir tout en passant au travers de ladite bande pour assurer une liaison fluidique entre le réservoir et la tubulure qui est emmanchée sur le connecteur.

Selon un mode de réalisation, illustré sur la figure 2A, le dispositif de fermeture de la manchette 1 comprend une ouverture oblongue 14 destinée à s'engager sur le connecteur 12.

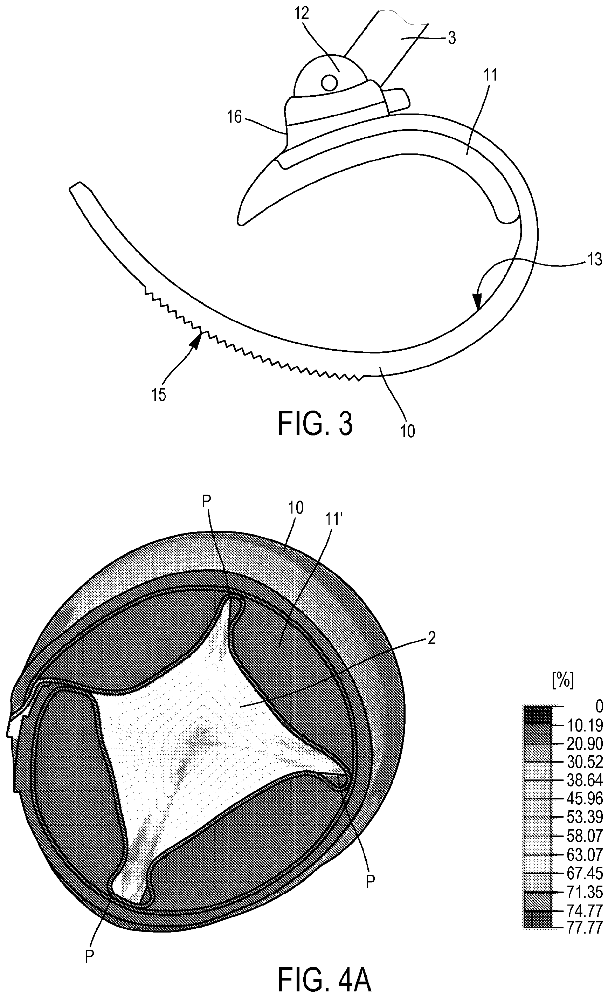

Selon un autre mode de réalisation, illustré sur la figure 3, la bande présente à une première extrémité une pluralité de crans 15 et à son autre extrémité, de préférence au niveau du connecteur 12, un dispositif d'attache 16 comprenant une fente adaptée pour permettre l'insertion de la première extrémité et munie d'un système de retenue n'autorisant un déplacement de la bande 10 que dans le sens de l'insertion (les crans s'opposant à un mouvement de traction visant à faire ressortir la bande de la fente). De manière avantageuse, les crans 15 se trouvent sur la face extérieure de la bande 10, afin de ne pas risquer d'éroder la partie comprimée du conduit. Les crans peuvent former partie intégrante de la bande ou être fabriqués par moulage puis collés sur la bande.

Naturellement, l'homme du métier pourrait choisir tout autre dispositif de fermeture sans pour autant sortir du cadre de la présente invention.

La réduction de la longueur du réservoir gonflable a trois effets avantageux principaux.

Un premier effet est une diminution de l'encombrement de la manchette, ce qui facilite son implantation dans des zones où l'espace disponible est limité. A l'état dégonflé préalable à l'implantation, la manchette se présente sous la forme d'une bande présentant une faible épaisseur, qui peut donc être insérée relativement facilement dans la zone disséquée autour du col vésical, contrairement à la manchette décrite dans le document WO 2013/165563, qui utilise la coopération d'une enclume solidaire de la surface intérieure de la bande et d'un marteau solidaire du réservoir pour comprimer le conduit anatomique. L'enclume et le marteau étant relativement rigides, ils présentent un encombrement non négligeable dans la zone d'implantation. Au contraire, dans la présente invention, comme cela apparaît clairement sur les figures 2B et 2C, lorsque la manchette est implantée autour du conduit anatomique, ledit conduit est en contact direct avec le réservoir 11 et avec la surface libre 13 de la bande qui sont des pièces souples, ce qui permet de minimiser l'épaisseur de la manchette et réduire le risque d'érosion des tissus anatomiques.

Un deuxième effet avantageux de l'invention est une réduction du volume de fluide nécessaire pour gonfler le réservoir afin d'obturer le conduit anatomique.

Par conséquent, le volume du récipient à volume variable contenu dans le boîtier reste limité, et l'autonomie de la source d'énergie n'est pas pénalisée.

Un troisième effet est une modification du mode d'action de la manchette sur le conduit anatomique.

La figure 4A présente le résultat d'une simulation par éléments finis des contraintes compressives appliquées au col vésical par une manchette selon l'état de la technique, telle qu'illustrée sur la figure 1A, pour une pression de fluide dans la manchette de 120 cm H₂O. On observe que la courbure de la manchette combinée à un gonflement du réservoir a pour effet de diviser le réservoir 11' en quatre poches qui s'étendent chacune sur environ un quart de la circonférence intérieure de la manchette. Or, le col vésical est pincé entre deux poches adjacentes, et est soumis, dans ces zones de pincement P, à des contraintes mécaniques locales bien supérieures aux contraintes auxquelles est soumis le reste du col. Ces contraintes localisées sont susceptibles d'endommager le col vésical, par exemple de l'atrophier.

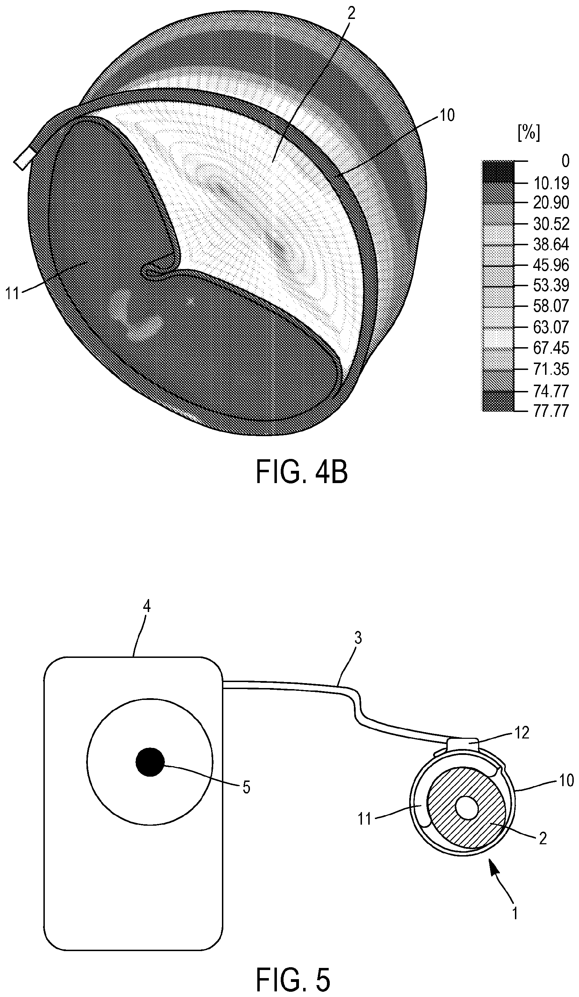

Sur les figures 4A et 4B, les niveaux de contraintes représentés avec des couleurs foncées correspondent aux contraintes élevées sur l'échelle de couleur figurant à droite de chaque simulation.

La figure 4B présente le résultat d'une simulation par éléments finis des contraintes compressives appliquées au col vésical par une manchette selon un mode de réalisation de l'invention, telle qu'illustrée sur la figure 2A, pour une pression de fluide dans la manchette de 120 cm H₂O. On observe que la réduction de la longueur du réservoir gonflable 11 a pour effet de supprimer les zones de pincement du col vésical, et de supprimer par conséquent les excès de contraintes subies localement par le col vésical. Le col vésical est donc moins susceptible de s'endommager.

La figure 5 illustre un système occlusif implantable comprenant un boîtier implantable comprenant une pompe, et une manchette occlusive telle que décrite ci-dessus, en liaison fluidique avec la pompe. La pompe est avantageusement l'un des dispositifs décrits dans le document WO 2016/083428.

Le boîtier 4 contient le récipient à volume variable, l'actionneur ainsi que le ou lesdits modules électroniques et, le cas échéant, la source d'énergie. Le boîtier contient un gaz, par exemple de l'air. Ledit boîtier doit être étanche pour éviter tout transfert de fluide ou de gaz de ou vers le milieu intracorporel. Le boîtier est en un matériau biocompatible et peut par exemple être réalisé en titane implantable et scellé par soudure laser. Un contrôle de l'étanchéité peut notamment être réalisé à l'hélium (par exemple, taux de fuite inférieur à 10⁻⁹ mbar.l/s d'hélium) pour s'assurer de l'étanchéité totale du boîtier pendant la période pour laquelle le système est implanté.

Le récipient à volume variable est relié à la manchette 1 par la tubulure 3.

De manière avantageuse, le boîtier comprend, dans une paroi délimitant le récipient à volume variable, un port de ponction 5 perforable par une aiguille et apte à se refermer de manière étanche après le retrait de l'aiguille, permettant d'injecter ou de retirer du fluide du récipient.

### REFERENCES

WO 2016/083428
WO 2013/165563

## Revendications

1. Manchette occlusive (1) pour obturer sélectivement un conduit anatomique (2), comprenant :
- une bande (10) adaptée pour entourer ledit conduit anatomique (2), pourvue d'un dispositif de fermeture adapté pour maintenir la bande enroulée sur elle-même sur une longueur déterminée (L₁₀),
- un réservoir gonflable (11) agencé sur une face intérieure de la bande (10), et
- un connecteur (12) agencé à une extrémité de la bande pour assurer une liaison fluidique entre le réservoir (11) et une tubulure (3),
ladite manchette étant **caractérisée en ce que** le réservoir gonflable (11) s'étend sur seulement une partie (L₁₁) de la longueur (L₁₀) de la bande (10), une autre partie (L₁₃) de ladite longueur (L₁₀) définissant une surface intérieure libre (13) de la bande configurée pour former une zone d'appui pour le conduit anatomique, de sorte que lorsque la bande (10) est maintenue enroulée sur elle-même par le dispositif de fermeture, le réservoir gonflable (11) est en vis-à-vis de ladite surface intérieure libre (13) de la bande.

2. Manchette selon la revendication 1, dans laquelle la longueur (L₁₁) du réservoir (11) est choisie de telle sorte que lorsque la bande est enroulée sur elle-même autour du conduit anatomique (2) et que le réservoir (11) est gonflé, le conduit anatomique est comprimé entre la paroi du réservoir (11) et la surface libre (13) de la bande, le réservoir (11) exerçant un effort de poussée du conduit anatomique (2) contre ladite surface libre (13) de la bande.

3. Manchette selon l'une des revendications 1 ou 2, dans laquelle le réservoir gonflable (11) et la surface intérieure libre (13) de la bande sont configurés pour être en contact direct avec le conduit anatomique lorsque la bande est enroulée autour dudit conduit anatomique (2).

4. Manchette selon l'une des revendications 1 à 3, dans laquelle le réservoir gonflable (11) s'étend sur une longueur (L₁₁) inférieure ou égale à 75% de la longueur (L₁₀) de la bande.

5. Manchette selon l'une des revendications 1 à 4, dans laquelle la longueur de la bande est comprise entre 30 et 110 mm, de préférence entre 70 et 110 mm.

6. Manchette selon l'une des revendications 1 à 5, dans laquelle la bande (10) est formée d'un textile enrobé d'un élastomère biocompatible.

7. Manchette selon l'une des revendications 1 à 6, dans laquelle le réservoir (11) est réalisé en un élastomère biocompatible.

8. Manchette selon l'une des revendications 6 ou 7, dans laquelle ledit élastomère biocompatible est du silicone.

9. Manchette selon l'une des revendications 1 à 8, dans laquelle le dispositif de fermeture comprend une ouverture (14) ménagée à une extrémité de la bande (10) et apte à s'engager autour du connecteur (12) pour maintenir la bande enroulée sur elle-même.

10. Manchette selon l'une des revendications 1 à 9, dans laquelle le dispositif de fermeture comprend une surface pourvue de crans (15) à une extrémité de la bande (10) et un dispositif d'attache (16) comprenant une fente pour l'insertion de ladite extrémité, configuré pour bloquer les crans à l'encontre d'un effort d'extraction de ladite extrémité.

11. Manchette selon la revendication 10, dans laquelle les crans (15) sont agencés sur la face extérieure de la bande (10).

12. Système occlusif implantable comprenant un boîtier implantable comprenant une pompe, et une manchette occlusive selon l'une des revendications 1 à 11, en liaison fluidique avec la pompe pour faire varier le volume de fluide dans le réservoir gonflable.

13. Système selon la revendication 12, consistant en un sphincter urinaire artificiel.

## Patentansprüche

1. Okklusionsmanschette (1) zum selektiven Verschließen eines Körpergangs (2), umfassend:
- ein Band (10), das dazu geeignet ist, den Körpergang (2) zu umgeben, und mit einer Schließvorrichtung versehen ist, die dazu geeignet ist, das über eine vorbestimmte Länge (L₁₀) um sich selbst aufgerollte Band festzuhalten,
- ein flexibles Reservoir (11), das auf einer Innenseite des Bandes (10) angeordnet ist, und
- ein Verbindungsstück (12), das an einem Ende des Bandes angeordnet ist, um eine Fluidverbindung mit dem Reservoir (11) und einem Schlauch (3) sicherzustellen,
wobei die Manschette **dadurch gekennzeichnet ist, dass** sich das flexible Reservoir (11) nur über einen Teil (L₁₁) der Länge (L₁₀) des Bandes (10) erstreckt, wobei ein anderer Teil (L₁₃) der Länge (L₁₀) eine freie innere Oberfläche (13) des Bandes definiert, die dazu konfiguriert ist, derart einen Stützbereich für den Körpergang zu bilden, dass wenn das Band (10) von der Schließvorrichtung um sich selbst aufgerollt gehalten wird, das flexible Reservoir (11) der freien inneren Oberfläche (13) des Bandes gegenübersteht.

2. Manschette nach Anspruch 1, wobei die Länge (L₁₁) des Reservoirs (11) derart gewählt wird, dass wenn das Band um den Körpergang (2) herum um sich selbst aufgerollt ist und das Reservoir (11) gefüllt wird, der Körpergang zwischen der Wand des Reservoirs (11) und der freien Oberfläche (13) des Bandes zusammengedrückt wird, wobei das Reservoir (11) eine Schubkraft des Körpergangs (2) auf die freie Oberfläche (13) des Bandes ausübt.

3. Manschette nach einem der Ansprüche 1 oder 2, wobei das flexible Reservoir (11) und die freie innere Oberfläche (13) des Bandes dazu konfiguriert sind, mit dem Körpergang in direktem Kontakt zu stehen, wenn das Band um den Körpergang (2) herum aufgerollt ist.

4. Manschette nach einem der Ansprüche 1 bis 3, wobei sich das flexible Reservoir (11) über eine Länge (L₁₁) erstreckt, die kleiner oder gleich 75 % der Länge (L₁₀) des Bandes ist.

5. Manschette nach einem der Ansprüche 1 bis 4, wobei die Länge des Bandes zwischen 30 und 110 mm, bevorzugt zwischen 70 und 110 mm, liegt.

6. Manschette nach einem der Ansprüche 1 bis 5, wobei das Band (10) aus einer Textilware gebildet ist, die mit einem körperverträglichen Elastomer überzogen ist.

7. Manschette nach einem der Ansprüche 1 bis 6, wobei das Reservoir (11) aus einem körperverträglichen Elastomer ausgebildet ist.

8. Manschette nach einem der Ansprüche 6 oder 7, wobei das körperverträgliche Elastomer Silikon ist.

9. Manschette nach einem der Ansprüche 1 bis 8, wobei die Schließvorrichtung eine Öffnung (14) umfasst, die an einem Ende des Bandes (10) eingerichtet ist und dazu geeignet ist, um das Verbindungsstück (12) herum in Eingriff zu kommen, um das um sich selbst aufgerollte Band festzuhalten.

10. Manschette nach einem der Ansprüche 1 bis 9, wobei die Schließvorrichtung eine Oberfläche, die mit Rastkerben (15) an einem Ende des Bandes (10) versehen ist, und eine Anbringungsvorrichtung (16), die einen Schlitz zum Einfügen des Endes umfasst und dazu konfiguriert ist, die Rastkerben gegen eine Auszugkraft des Endes zu blockieren, umfasst.

11. Manschette nach Anspruch 10, wobei die Rastkerben (15) auf der Außenseite des Bandes (10) angeordnet sind.

12. Implantierbares Okklusionssystem, umfassend ein implantierbares Gehäuse, das eine Pumpe umfasst, und eine Okklusionsmanschette nach einem der Ansprüche 1 bis 11 in Fluidverbindung mit der Pumpe, um das Fluidvolumen in dem flexiblen Reservoir zu variieren.

13. System nach Anspruch 12, bestehend aus einem künstlichen Schließmuskel.

## Claims

1. Occlusive cuff (1) for selectively occluding an anatomical duct (2), comprising:
- a band (10) suited to surrounding said anatomical duct (2), provided with a closing device suited to maintaining the band wound upon itself over a determined length (L₁₀),
- an inflatable reservoir (11) arranged on an inner face of the band (10), and
- a connector (12) arranged at one end of the band to ensure a fluid connection between the reservoir (11) and a tubing (3),
said cuff being **characterised in that** the inflatable reservoir (11) extends over only a part (L₁₁) of the length (L₁₀) of the band (10), another part (L₁₃) of said length (L₁₀) defining a free inner surface (13) of the band configured to form a bearing zone for the anatomical duct, such that when the band (10) is maintained wound upon itself by the closing device, the inflatable reservoir (11) is opposite said free inner surface (13) of the band.

2. Cuff according to claim 1, wherein the length (L₁₁) of the reservoir (11) is chosen such that when the band is wound upon itself around the anatomical duct (2) and when the reservoir (11) is inflated, the anatomical duct is compressed between the wall of the reservoir (11) and the free surface (13) of the band, the reservoir (11) exerting a push force of the anatomical duct (2) against said free surface (13) of the band.

3. Cuff according to one of claims 1 or 2, wherein the inflatable reservoir (11) and the free inner surface (13) of the band are configured to be in direct contact with the anatomical duct when the band is wound around said anatomical duct (2).

4. Cuff according to one of claims 1 to 3, wherein the inflatable reservoir (11) extends over a length (L₁₁) less than or equal to 75% of the length (L₁₀) of the band.

5. Cuff according to one of claims 1 to 4, wherein the length of the band is comprised between 30 and 110 mm, preferably between 70 and 110 mm.

6. Cuff according to one of claims 1 to 5, wherein the band (10) is formed of a textile coated with a biocompatible elastomer.

7. Cuff according to one of claims 1 to 6, wherein the reservoir (11) is made of a biocompatible elastomer.

8. Cuff according to one of claims 6 or 7, wherein said biocompatible elastomer is silicone.

9. Cuff according to one of claims 1 to 8, wherein the closing device comprises an opening (14) formed at one end of the band (10) and able to engage around the connector (12) to maintain the band wound upon itself.

10. Cuff according to one of claims 1 to 9, wherein the closing device comprises a surface provided with teeth (15) at one end of the band (10) and an attachment device (16) comprising a slot for the insertion of said end, configured to block the teeth against an extraction force of said end.

11. Cuff according to claim 10, wherein the teeth (15) are arranged on the outer face of the band (10).

12. Implantable occlusive system comprising an implantable housing comprising a pump, and an occlusive cuff according to one of claims 1 to 11, in fluid connection with the pump to vary the volume of fluid in the inflatable reservoir.

13. System according to claim 12, consisting of an artificial urinary sphincter.
